# EUROPEAN PATENT SPECIFICATION

(11) **EP 4 240 842 B1**
(45) Date of publication and mention of the grant of the patent: **18.12.2024**
(21) Application number: 21807261.9
(22) Date of filing: 02.11.2021
(51) Int. Cl.: C12N 15/10, C12Q 1/6806

(54) **TARGETED SEQUENCING METHOD AND KIT THEREOF FOR DETECTING GENE ALTERATION**
GEZIELTE SEQUENZIERUNGSVERFAHREN UND KIT DAFÜR ZUM NACHWEIS VON GENVERÄNDERUNG
PROCÉDÉ DE SÉQUENÇAGE CIBLÉ ET KIT ASSOCIÉ POUR DÉTECTER UNE ALTÉRATION GÉNÉTIQUE

(30) Priority: 03.11.2020 US 202063108932 P; 26.02.2021 US 202163153956 P
(43) Date of publication of application: 13.09.2023
(73) Proprietor: ACT Genomics (IP) Limited, Shatin, Hong Kong (HK)
(72) Inventor: LAI, Yi-Hsuan, Taipei City, Taiwan 114 (TW); CHEN, Yu-Ling, Taipei City, Taiwan 114 (TW); HSU, An, Taipei City, Taiwan 114 (TW); LIN, Pei-Yi, Taipei City, Taiwan 114 (TW); CHEN, Hua-Chien, Taipei City, Taiwan 114 (TW); CHEN, Shu-Jen, Taipei City, Taiwan 114 (TW)
(74) Representative: dompatent von Kreisler Selting Werner - Partnerschaft von Patent- und Rechtsanwälten mbB
(86) International application number: PCT/IB2021/060130
(87) International publication number: WO 2022/097021

(56) References cited:
- WO-A1-2011/070155
- WO-A1-2016/138490
- WO-A1-2017/048993
- WO-A1-2017/087873
- WO-A1-2017/173328
- WO-A1-2018/089550
- WO-A1-2018/218222
- WO-A1-2018/229514
- WO-A1-2020/072380
- WO-A1-2020/118200
- WO-A1-2020/136438
- WO-A1-2021/214307
- WO-A1-2021/247593
- CN-A- 108 103 175

## Description

### CROSS REFERENCE TO RELATED APPLICATIONS

This application claims priority to U.S. Application No. 2020/63108932, filed November 3, 2020, and U.S. Application No. 2021/63153956, filed February 26, 2021.

### FIELD

This application is related to the fields of molecular diagnostics, cancer genetics, and molecular biology. More particularly, this application relates to a method and a kit for detecting a gene fusion event.

### REFERENCE TO SEQUENCE LISTING SUBMITTED ELECTRONICALLY

This application contains a sequence listing, which is submitted electronically via EFS-Web as an ASCII formatted sequence listing with a file name "066997-1WO1 Sequence Listing" and a creation date of October 12, 2021 and having a size of 7.1 kb. The sequence listing submitted via EFS-Web is part of the specification and is herein incorporated by reference in its entirety.

### BACKGROUND

Genetic alterations are collectively referred to changes in normal DNA sequences, such as gene fusion, point mutation, insertion, deletion, amplification, and rearrangement. When the DNA sequence is altered, dysfunctional or abnormally activated proteins may be produced, resulting in diseases such as cancer. Thus, detecting genetic alterations is a critical step to improve disease surveillance and to provide subsequent treatments.

Gene fusion is one of the genetic alterations that plays an important role in tumorigenesis. Two originally separate and functionally distinct genes can fuse together as a result of translocation, interstitial deletion, or chromosomal inversion, generating various types of gene fusion. One prevalent type of gene fusion includes a kinase gene fused to a highly expressed partner gene, resulting in tumor formation, since the fused gene can now result in excessive amount of proteins with potentially aberrant activity. Because many gene fusions lead to cancer, fusion genes can be used as the targets for drug development. Therefore, it is important to detect all possible gene fusions in order to identify patients who can benefit from these targeted therapies. For example, a method for one-time detection of EML4-ALK, ROS1 and RET fusion gene mutation through library construction and sequencing by starting from 1-2000 fresh tissue cells, 10pg-20ng of extracted total RNA or plasma free RNA, carrying out reverse transcription on mRNA containing fusion type information under the action of ALK, ROS1, RET specific primer and reverse transcriptase and obtaining a first strand of cDNA, then adding a segment of linker sequence (with molecular label) at the 3' end of the cDNA by a template conversion technology and carrying out cDNA double-strand generation, and then adding Illumina library joints at the two ends of the cDNA while carrying out exponential amplification by taking the linker segment as a primer anchoring site so as to obtain a high-quality cDNA library meeting the downstream analysis requirements within 3 hours is disclosed in CN 108103175 A.

Currently, many platforms for detecting gene fusions are based on the known sequences of the gene fusions. For example, in traditional PCR, known sequences are required to properly design target-specific forward and reverse primers for the amplification process. However, it is difficult to use such PCR techniques to detect all possible gene fusions, because many of which are not identified previously and have unknown sequences. Although methods such as 5'/3' rapid amplification of cDNA ends (RACE) can be used to overcome this difficulty, clinical samples with low quality and quantity of the fusion genes still pose a limitation to those methods. A method of ordering nucleic acid molecule fragment sequences derived from a pool of potentially diverse RNA molecules comprising optionally reverse transcribing the RNA molecules to provide a pool of cDNA molecules, segregating nucleic acids from said template RNA or cDNA pool, selecting for potentially different templates with a distinctive nucleic acid feature shared by the segregated templates, thereby providing at least a first subpool of nucleic acids, optionally once or more further segregating nucleic acids from said template RNA or cDNA, selectively segregating nucleic acids with a different distinctive nucleic acid feature, thereby providing one or more further subpool(s) of nucleic acids, generating fragments of said segregated nucleic acid molecules by fragmenting or obtaining fragment copies of said segregated nucleic acid molecules, wherein the fragments of each sub-pool or combined subpools remain separable from fragments of other subpools or other combined subpools by physically separating the subpools or by attaching a label to the fragments of the subpools, with the label identifying a subpool, or determining a partial sequence of said segregated nucleic acid molecule and preferably aligning at least two sequences or partial sequences to a joined sequence is described in WO 2011/070155 A1. WO 2018/218222 discloses template-switch-based addition of a universal adaptor sequence to which a vessel barcode hybridizes for dual barcoding of cDNA.

Targeted next-generation sequencing (NGS)-based platforms are ideal in clinical practice and can be distinguished by hybrid capture (e.g. FoundationOne94,97) and amplicon-based approaches (e.g. Archer FusionPlex Solid Tumor Panel120,121) for target enrichment. Amplicon-based approaches target genome or transcriptome sequences by using gene specific primers for polymerase chain reaction (PCR) amplification of target sequences during library construction. For multiplex PCR, a universal sequence is added to the target sequence by PCR amplification with a gene-specific primer at one end and a universal primer at the other end. RNA-based NGS technologies are able to detect 3' fusion partner by using universal and gene-specific primers in multiplexed assays (e.g. OmniFusion RNA Lung Cancer Panel) or detect any known or unknown 5' or 3' fusion partner by adapter ligation technology, such as the Archer FusionPlex NGS assay. However, the performance of detecting novel fusions or known fusions with novel breakpoints is affected by library preparation technology, exon coverage and detection efficiency.

Hence, there is an unmet medical need for an accurate and highly sensitive gene fusion detection method, which is capable of identifying gene fusions with unknown sequences in low quality and quantity clinical samples.

### SUMMARY OF THE DISCLOSURE

The invention is set out in the independent claims. Preferred embodiments are reflected in the dependent claims.

The present application provides a method for detecting a gene alteration, comprising steps of
(a) mixing a target RNA obtained from the biological sample, a template-switching oligo, a reverse transcriptase, and a reverse transcription (RT) primer in a mixture, wherein the template switch oligo comprises a first universal primer sequence and the RT primer comprises a second universal primer sequence;
(b) subjecting the mixture to a condition under which reverse transcription occurs to provide a target DNA complementary to the target RNA,
(c) amplifying the target DNA with a first pair of primers to obtain a first PCR product, wherein the first pair of primers comprise a gene specific primer and a universal primer, the gene specific primer comprises a 5'-end first or second adapter sequence and a 3'-end sequence that hybridizes to a target gene under stringent conditions, the universal primer comprises a 5'-end first or second adapter sequence and a 3'-end sequence comprising the first or second universal primer sequence, and the first and second adapter sequences are different from each other;
(d) amplifying the first PCR product with a second pair of primers to obtain a second PCR product, wherein each of the second pair of primers comprises a 5'-end third or fourth adapter sequence and a 3'-end sequence that hybridizes to the first or second adapter sequence, respectively, and the third and fourth adapter sequences are different from each other; and
(e) analyzing the second PCR product to detect the presence of the gene alteration, preferably, the second PCR product is analyzed by sequencing analysis utilizing at least one adapter sequence.

According to the above, the RT primer comprises a linear structure having at least 5 random nucleotides.

According to the above, the RT primer comprises a stem-loop structure and an overhang structure having at least 5 random nucleotides.

According to the above, the stem-loop structure is a hairpin stem-loop structure or a Y shape stem-loop structure.

According to the above, the stem-loop structure comprises a barcode sequence.

According to the above, the stem-loop structure is at least the length of the second universal primer sequence.

According to the above, the second universal primer sequence is less than 30 bp in length.

According to the above, the stem of the stem-loop structure is 8 bp in length.

According to the above, at least one of the first adapter sequence or the second adapter sequence includes a barcode sequence.

According to the above, the target DNA is at least 100 bp in length.

According to the above, the target DNA is 100 bp to 4000 bp in length.

According to the above, the target DNA is 100 bp to 500 bp in length.

According to the above, the target DNA is amplified by multiplex PCR with at least two gene specific primers in step (c).

According to the above, the first PCR product is generated and separated by 3' or 5' direction of the gene specific primer.

According to the above, the gene specific primer hybridizes to the target DNA within a distance of at least 25 bp from a fusion junction boundary.

According to the above, the one or more gene specific primers are selected from the group consisting of SEQ ID NOs:11, 12, 14-35 and any complementary sequence thereof.

According to the above, the universal primer is selected from the group consisting of SEQ ID NOs:1-10 and any complementary sequence thereof.

According to the above, the gene alteration is a gene fusion comprising a sequence of a known gene selected from the group consisting of ABL1, AKT3, ALK, ARV7, BCR, BRAF, CD74, EGFR, ERBB2, ERBB4, ERG, ESR1, ETV1, ETV4, ETV5, ETV6, EZR, FGFR1, FGFR2, FGFR3, KIT, KMT2A, MET, NRG1, NRG2, NTRK1, NTRK2, NTRK3, NUTM1, PDGFRA, PDGFRB, PIK3CA, RAF1, RARA, RET, ROS1, RSPO2, SDC4, SLC34A2 and TMPRSS2.

According to the above, the biological sample is from a solid tumor.

According to the above, the biological sample is a Formalin-Fixed Paraffin-Embedded (FFPE) tissue sample.

According to the above, the second PCR product is analyzed by a next generation sequencing.

In one aspect, the present application also provides a kit for detecting a gene alteration in a biological sample, comprising
(a) a template switch oligo comprising a first universal primer;
(b) a reverse transcription (RT) primer comprising a stem-loop structure and an overhang structure of at least 5 random tail nucleotides, wherein the RT primer comprises a second universal primer;
(c) one or more gene specific primers each comprising a first or second adapter sequence and universal primers each comprising a first or second adapter sequence, respectively, wherein the first adapter sequence of the gene specific primer is different from the second adapter sequence of the universal primer or the second adapter sequence of the gene specific primer is different from the first adapter sequence of the universal primer;
(d) a primer pair complementing the first and second adapters, respectively, wherein each primer of the primer pair comprises a third or fourth adapter sequence, respectively, and wherein the third and fourth adapter sequences are different from each other;
(e) a reverse transcriptase;
(f) a DNA polymerase; and
(g) deoxy-ribonucleoside triphosphate (dNTP).

Also provided is a kit for detecting a gene alteration in a biological sample, comprising:
(a) a template switch oligo comprising a first universal primer;
(b) a reverse transcription (RT) primer comprising a linear structure of at least 5 random tail nucleotides, wherein the RT primer comprises a second universal primer;
(c) one or more gene specific primers each comprising a first or second adapter sequence and universal primers each comprising a first or second adapter sequence, respectively, wherein the first adapter sequence of the gene specific primer is different from the second adapter sequence of the universal primer or the second adapter sequence of the gene specific primer is different from the first adapter sequence of the universal primer;
(d) a primer pair complementing the first and second adapters, respectively, wherein each primer of the primer pair comprises a third or fourth adapter sequence, respectively, and wherein the third and fourth adapter sequences are different from each other;
(e) a reverse transcriptase;
(f) a DNA polymerase; and
(g) deoxy-ribonucleoside triphosphate (dNTP).

According to the above, the second universal primer sequence is less than 30 bp in length.

According to the above, the stem of the stem loop primer is 8 bp in length.

According to the above, the overhang structure is a random hexamer with a length of 5 to 10 nucleotides.

According to the above, the kit further comprises at least one labeled dNTP, wherein the dNTP is labeled with a biotin group, Digoxigenin (DIG) or other molecules.

### BRIEF DESCRIPTION OF THE DRAWINGS

One or more embodiments are illustrated by ways of example, and not by limitation, in the figures of the accompanying drawings, wherein elements are having the same reference numeral designations represent like elements throughout. The drawings are not to scale unless otherwise disclosed.
Figs. 1(a)-(c) are schematic illustrations of a method for detecting a gene alteration according to one embodiment of the application.
Fig. 2 is a schematic illustration of the different RT primer designs of the application.
Fig. 3 is a schematic illustration of the other different RT primer designs of useful for the invention.
Figs. 4 (a)-(b) show the relative fluorescence unit (RFU) values of the PCR products with different sizes obtained by using different RT primers.
Figs. 5 (a)-(b) show the relative fluorescence unit (RFU) values of the PCR products with different sizes obtained by using RT primers with different lengths of the random tail nucleotides.

The drawings are only schematic and are non-limiting. In the drawings, the size of some of the elements may be exaggerated and not drawn to scale for illustrative purposes. The dimensions and the relative dimensions do not necessarily correspond to actual reductions to the practice of the disclosure.

### DETAILED DESCRIPTION

The making and using of the embodiments of the disclosure are discussed in detail below. It should be appreciated, however, that the embodiments provide many applicable inventive concepts that can be embodied in a wide variety of specific contexts. The specific embodiments discussed are merely illustrative of specific ways to make and use the embodiments and do not limit the scope of the disclosure.

Various publications, articles, patents and patent applications are cited or described in the background and throughout the specification. Discussion of documents, acts, materials, devices, articles or the like which has been included in the present specification is for the purpose of providing context for the present application. Such discussion is not an admission that any or all of these matters form part of the prior art with respect to any inventions disclosed or claimed.

Unless defined otherwise, all technical and scientific terms used herein have the same meaning as commonly understood by a person skilled in the art to which this disclosure belongs. As used herein, the singular forms "a", "an", and "the" include plural referents unless the context clearly dictates otherwise.

In this disclosure, gene alteration comprises gene fusion, point mutation, insertion, deletion, amplification, or rearrangement.

The term "gene fusion" or "fusion gene" refers to the gene alteration including a hybrid gene formed from two previously independent genes or portions thereof or a particular gene formed from at least two independent exons thereof. In this disclosure, MET exon 14 skipping is considered one fusion type. For example, MET gene fusion is a fusion between at least a part of MET gene and a part of a highly expressed partner gene. MET gene fusion is also caused by aberrant splicing, which leads to fusion between exon 13 and exon 15 of the MET gene (MET exon 14 skipping). A fusion gene is formed when a first gene or a portion of the first gene on a chromosome is fused to a second gene or a portion of the second gene on the same or a different chromosome as a result of gene alteration, such as translocation, interstitial deletion, or chromosomal inversion. "Gene fusion" is also referred to as "gene translocation" or "gene rearrangement." For example, when an NTRK gene or a portion thereof is part of a gene fusion, such gene fusion is called "NTRK gene fusion" or "NTRK fusion."

The gene at the 5' end of a fusion gene is referred to as the "5' gene" of the fusion gene, and the gene at the 3' end of the fusion gene is referred to as the "3' gene" of the fusion gene. The fusion gene has a "fusion gene breakpoint," which is the site where the genes fuse. "Fusion gene breakpoint" as used herein can be used interchangeably with "breakpoint". Fusion gene breakpoint is located in a region of the fusion gene defined by a fusion junction sequence, which encompasses the sequence from the 5' gene and the sequence from the 3' gene. Different fusion gene breakpoints can lead to different "fusion types." The fusion between two specific genes can result in different fusion genes, because the fusion breakpoint can occur anywhere within the fusion genes. For example, the fusion between the first exon of a first gene and the second exon of a second gene is one fusion type, whereas the fusion between the third exon of the first gene and the first exon of the second gene is another fusion type.

Gene fusions can be detected by identifying a fusion junction in a DNA or in an RNA transcript of that DNA. As used herein, a "fusion type" refers to a unique fusion junction sequence present in an RNA transcript of a fusion gene. In other words, fusion genes of two specific genes are considered the same fusion type when they contain the same fusion junction sequence in their RNA transcript, even though the fusion genes may have different fusion junction sequences in their DNA sequences. Fusion genes can be the same fusion type when the fusions between the two specific genes occur at different sites within the same intronic region. For example, a fusion between exon 3 of gene A and exon 5 of gene B can have a DNA fusion region containing a small portion of the intron extended from exon 3 between exons 3 and 4 of gene A and a large portion of the intron extended from exon 5 between exons 4 and 5 of gene B. Alternatively, such fusion can have a DNA fusion region containing a large portion of the intron extended from exon 3 between exons 3 and 4 of gene A and a small portion of the intron extended from exon 5 between exons 4 and 5 of gene B. These two fusions, though having different DNA fusion junctions, are considered the same "fusion type" because the RNA transcripts generated from the two fusions genes have the same fusion junction sequence.

As used herein, the terms "reverse transcription primer" or "RT primer" refer to a DNA primer that contains a universal primer sequence and a linear or a stem-loop with an overhang structure of at least 5 random tail nucleotides. The RT primer binds to the 3' end of an RNA sequence to make the first strand cDNA using reverse transcription.

As used herein, the term "template switch oligo" or "TS oligo" or "TSO" refers to an oligo nucleotide sequence that contains, from the 5'-end to 3'-end, a universal primer sequence and a sequence complementary to the non-templated nucleotides at the 3' end of a synthesized cDNA. When cDNA is synthesized by a transcriptase using an RNA template, upon reaching the 5' end of the RNA template, the terminal transferase activity of the reverse transcriptase adds a few additional non-templated nucleotides (e.g., mostly deoxycytidine when Moloney murine leukemia virus (MMLV) reverse transcriptase is used) to the 3' end of the newly synthesized cDNA strand. These non-templated nucleotides function as an anchoring site for the TS oligo. Upon base pairing between the TS oligo and the non-templated nucleotides, the reverse transcriptase "switches" template strands, from the RNA to the TS oligo, and continues replication to the 5' end of the TS oligo. By doing so, the resulting cDNA contains the complete 5' end of the transcript, and the universal primer sequences of choice are added to the reverse transcription product. The TS oligos can be synthesized as an example by a method: 50 ng of RNA fragments were denatured at 65° C. for 5 minutes in the presence of 3 µM of random hexamer and 2 mM of dNTP in 14 µl, followed by immediate incubation on ice for 3 minutes, and then 4 µM of a template switching primer, reverse transcription buffer (50 mM Tris-HCl, pH 8.3 at 25° C., 75 mM KCl, 3 mM MgCl₂, 10 mM DTT) and 200 unites of SMARTScribe^{™} Reverse Transcriptase (TaKaRa, catalog number 639538) were added into the reaction, and the total volume of the reaction was 20 µl, and the reverse transcription and template switching reaction was carried out for 10 minutes at 8° C. followed by 80 minutes at 42° C, and the sequence of the template switching primer is 5' Biotin-TTC AGA CGT GTG CTC TTC CGA TCT rGrGrG 3' (made by Integrated DNA Technologies), which is described in U.S. patent application publication No. 2021/0180051("METHODS AND SYSTEMS TO AMPLIFY SHORT RNA TARGETS").

As used herein, the term "gene specific primer" refers to a DNA primer that is designed to bind specifically to a target DNA sequence of a gene of interest. In some embodiments of the application, the gene specific primer binds specifically to a DNA fragment of a fusion gene.

As used herein, the term "universal primer" refers to a DNA primer that is designed to amplify any DNA including the nucleotide sequence of the universal primer.

As used herein, the terms "adapter", "first adapter" "second adapter", "third adapter" and "fourth adapter" refer to a DNA adapter that is designed to add to the cDNA a varietal tag, a barcode, to make the sequencing library or includes the platform-specific sequences for recognition by the sequencer.

Each of the genes described herein correspond to a "gene name (or symbol)" listed in the NCBI gene database (www.ncbi.nlm.nih.gov/gene/). The NCBI gene database therefore can be used to identify the sequence of a gene or synonyms of the gene name.

In the present disclosure, a method for detecting a gene alteration is provided. The method comprises the steps of:
(a) mixing a target RNA obtained from the biological sample, a template-switching oligo, a reverse transcriptase, and a reverse transcription (RT) primer in a mixture, wherein the template switch oligo comprises a first universal primer sequence and the RT primer comprises a second universal primer sequence;
(b) subjecting the mixture to a condition under which reverse transcription occurs to provide a target DNA complementary to the target RNA,
(c) amplifying the target DNA with a first pair of primers to obtain a first PCR product, wherein the first pair of primers comprise a gene specific primer and a universal primer, the gene specific primer comprises a 5'-end first or second adapter sequence and a 3'-end sequence that hybridizes to a target gene under stringent conditions, the universal primer comprises a 5'-end first or second adapter sequence and a 3'-end sequence comprising the first or second universal primer sequence, and the first and second adapter sequences are different from each other;
(d) amplifying the first PCR product with a second pair of primers to obtain a second PCR product, wherein each of the second pair of primers comprises a 5'-end third or fourth adapter sequence and a 3'-end sequence that hybridizes to the first or second adapter sequence, respectively, and the third and fourth adapter sequences are different from each other; and
(e) analyzing the second PCR product to detect the presence of the gene alteration, preferably, the second PCR product is analyzed by sequencing analysis utilizing at least one adapter sequence.

In some embodiments, RNA is prepared from a biological sample. The biological sample may be any sample obtained from an animal and a human subject. Examples of the biological samples include a formalin-fixed paraffin-embedded (FFPE) tissue section, blood, plasma, or cells. In some embodiments, the biological sample originates from a cancer patient. In some embodiments, the biological sample originates from a carcinoma, a sarcoma, a lymphoma, a leukemia, or a myeloma. In some embodiments, the biological sample originates from a patient with brain cancer, breast cancer, colon cancer, endocrine gland cancer, esophageal cancer, female reproductive organ cancer, head and neck cancer, hepatobiliary system cancer, kidney cancer, lung cancer, mesenchymal cell neoplasm, prostate cancer, skin cancer, stomach cancer, tumor of exocrine pancreas and urinary system cancer.

Preparation of total RNA from the biological sample can be carried out by various methods known in the art. One typical procedure is RNA extraction with organic solvents such as phenol/chloroform and precipitation by centrifugation. There are also commercially available kits for RNA isolation or purification. Once the RNA is obtained, a reverse transcriptase is used along with four kinds of deoxyribonucleoside triphosphates (dNTP, including dATP, dCTP, dTTP, and dGTP) to generate cDNA from the template RNA, a process called reverse transcription. The reverse transcription may be conducted using SuperScript cDNA synthesis kit (Cat No: 11754050, Invitrogen).

In some embodiment, this method can detect a genetic gene alteration where its sequence is known. In some embodiment, this method can detect a genetic alteration where at least one end of its sequence is known. In some embodiment, this method can detect a known fusion gene. In some embodiment, this method can detect a gene fusion where a known gene fuses with an unknown gene. In some embodiment, this method detects a gene fusion that the 5' partner is known and the 3' partner is unknown. In some embodiment, this method detects a series of gene fusions that a same known partner fuses with a plurality of different unknown partner. In some embodiment, this method can detect multiple gene fusions that a plurality of different known partner fuse with a plurality of different unknown partner.

In some embodiment, the second PCR product in the step (d) of this method can be isolated by solid-phase nucleic acid extraction, such as anion-exchange material purification or magnetic bead based nucleic acid purification.

In some embodiments, at least one of the first adapter sequence or the second adapter sequence includes a barcode sequence.

In some embodiments, the target DNA is at least 100 bp in length.

In some embodiments, the target DNA is 100 bp to 4000 bp in length.

In some embodiments, the target DNA is 100 bp to 500 bp in length.

In some embodiments, the disclosed method further includes a step of amplifying the target DNA by multiplex PCR with at least two gene specific primers in step (c).

In some embodiments, the first PCR product is generated and separated by 3' or 5' direction of the gene specific primer.

In some embodiments, the gene specific primer hybridizes to the target DNA within a distance of at least 25 bp from a fusion junction boundary.

In some embodiments, the gene specific primer is selected from the group consisting of SEQ ID NOs: 11, 12, 14-35 and any complementary sequence thereof.

In some embodiments, the universal primer is selected from the group consisting of SEQ ID NOs: 1-10 and any complementary sequence thereof.

In other embodiments, the gene alteration is a gene fusion comprising a sequence of a known gene selected from the group consisting of ABL1, AKT3, ALK, ARV7, BCR, BRAF, CD74, EGFR, ERBB2, ERBB4, ERG, ESR1, ETV1, ETV4, ETV5, ETV6, EZR, FGFR1, FGFR2, FGFR3, KIT, KMT2A, MET, NRG1, NRG2, NTRK1, NTRK2, NTRK3, NUTM1, PDGFRA, PDGFRB, PIK3CA, RAF1, RARA, RET, ROS1, RSPO2, SDC4, SLC34A2 and TMPRSS2.

In some embodiment, the presence of the genetic alteration is identified from sequencing the second PCR product. In some embodiment, sequencing the second PCR product can be performed by Sanger sequencing, next-generation sequencing, or pyrosequencing. In some embodiment, the sequencing results from the second PCR product is further conducted by sequence alignment to identified the genetic alteration. In some embodiment, the sequence alignment may need a first or a second alignment. The first or the second alignment can be performed using one of several alignment algorithms or software, for example, Blastn, BLAT, BWN, BreakDancer, Burrows-Wheeler Aligner (BWA), BWA-MEM, BWA-SW, Bowtie, Stampy, Torrent Mapping Alignment Program (TMAP), or TopHat.

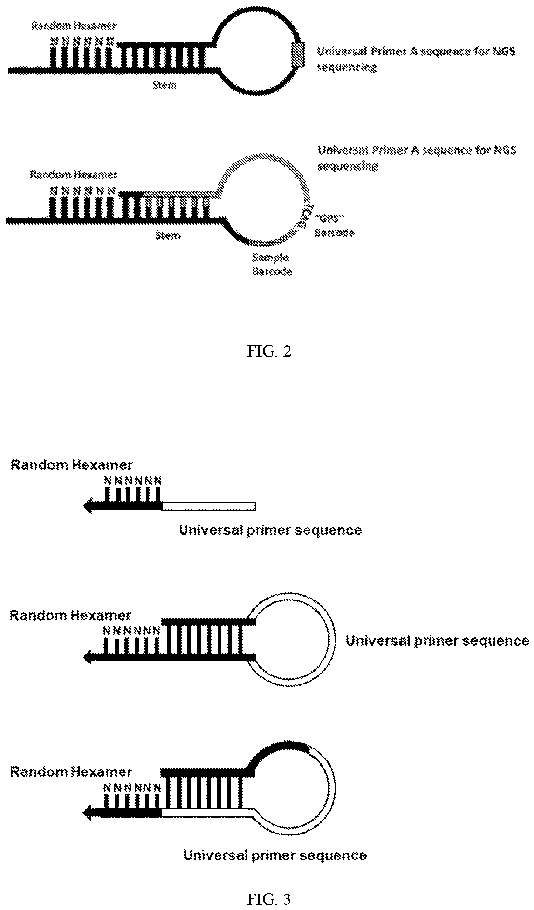

In some embodiment, this method uses a RT primer and a template switch oligo to perform universal primer extension of the target DNA. In some embodiment, the template switch oligo binds to 5' end and the RT primer binds to 3' end of any gene alteration sequences. In some embodiment, the RT primer comprises a stem-loop structure and an overhang structure having at least 5 random nucleotides. In some embodiment, the RT primer comprises a concatenation of the second universal primer sequence with at least 5 random tail nucleotides. In some embodiment, the stem-loop structure is a hairpin stem-loop structure or a Y shape stem-loop structure. In some embodiment, the overhang sequence is a random hexamer, a random sequence of about 5 to 10 contiguous nucleic acids. In some embodiment, the stem-loop structure comprises a barcode sequence. In some embodiment, the stem-loop structure is at least the length of the second universal primer sequence. In some embodiment, the RT primer comprises a second universal primer that is less than 30 bp in length. In some embodiment, the stem of the stem-loop primer is 8 bp in length. In some embodiment, the stem-loop structure comprises a universal primer, "GPS" barcode and sample barcode sequences (Fig. 2).

In some embodiment, the target DNA is synthesized with dNTP comprising at least one labeled dNTP. In some embodiment, the ratio of unlabeled dNTP to labeled dNTP for synthesizing the target DNA is in a range of 4:1 to 7:1. In some embodiment, the dNTP is labeled with biotin or other functional group. In some embodiment, the dNTP is labeled with biotin or other functional group through a linker. In some embodiment, the linker locates at a site of dNTP which cannot form the DNA hydrogen bond. In some embodiment, the linker is a carbon chain. In some embodiment the linker is a 16 carbon chain (16C). In some embodiment, the labeled dNTP comprises a biotin-16C-dCTP. In some embodiment, the dNTP labeled with biotin or other functional group is capable of connecting to streptavidin conjugated magnetic bead or other beads. In some embodiment, the target DNA labeled with biotin or other functional group is capable of connecting to streptavidin conjugated magnetic bead or other beads. In some embodiment, streptavidin conjugated magnetic bead or other beads can be attracted by a magnetic field. In some embodiment, the dNTP is labeled with Digoxigenin (DIG). In some embodiment, the dNTP labeled with DIG is capable of connecting to anti-DIG antibody that is commonly conjugated with horseradish peroxidase (HRP), alkaline phosphatase (AP) or a fluorescent dye. In some embodiment, the target DNA labeled with DIG is capable of connecting to anti-DIG antibody that is commonly conjugated with HRP, AP or a fluorescent dye.

As used herein, "a molecule" refers to a material that is capable of being labeled on the dNTP or the target DNA, such as biotin, other functional groups, or DIG. "A bait" refers to another material that is capable of connecting to the molecule, in order to capture the target DNA, such as streptavidin conjugated magnetic bead, other beads or anti-DIG antibody.

In some embodiment, the target DNA can be isolated by molecularly specific binding. In some embodiment, the target DNA can be isolated by the following step: (1) connecting the target DNA labeled with the molecule to the bait; (2) capturing the bait connecting to the target DNA; (3) washing any reagents away except the target DNA. In some embodiment, the target DNA can be isolated by the following step: (1) connecting the target DNA labeled with biotin or other functional group to streptavidin conjugated magnetic bead or other beads; (2) providing the magnetic field to capture the streptavidin conjugated magnetic bead or other beads connecting to the target DNA; (3) washing all reagents away besides the first stand DNA. In some embodiment, the target DNA can be isolated the following step: (1) modifying anti-DIG antibody on a solid phase; (2) connecting the target DNA labeled with DIG to anti-DIG antibody on a solid phase; (3) washing all reagents away besides the first stand DNA.

In the present disclosure, a kit for detecting a gene alteration is provided. In certain embodiments, the kit comprises
(a) a concatenation of a template switch oligo with a first universal primer;
(b) a reverse transcription (RT) primer comprising a stem-loop structure and an overhang structure of an at least 5 random tail nucleotides, and the RT primer comprises a second universal primer;
(c) one or more gene specific primers each comprising a first or second adapter sequence and universal primers each comprising a first or second adapter sequence, respectively, wherein the first adapter sequence of the gene specific primer is different from the second adapter sequence of the universal primer or the second adapter sequence of the gene specific primer is different from the first adapter sequence of the universal primer;
(d) a primer pair complementing the first adapters, wherein each primer of the primer pair comprises a second adapter, and wherein the second adapter sequences are different from each other;
(e) a reverse transcriptase;
(f) a DNA polymerase; and
(g) deoxy-ribonucleoside triphosphate (dNTP).

In other embodiments, the kit comprises
(a) a template switch oligo comprising a first universal primer;
(b) a reverse transcription (RT) primer comprising a linear structure of at least 5 random tail nucleotides, wherein the RT primer comprises a second universal primer;
(c) one or more gene specific primers each comprising a first or second adapter sequence and universal primers each comprising a first or second adapter sequence, respectively, wherein the first adapter sequence of the gene specific primer is different from the second adapter sequence of the universal primer or the second adapter sequence of the gene specific primer is different from the first adapter sequence of the universal primer;
(d) a primer pair complementing the first and second adapters, respectively, wherein each primer of the primer pair comprises a third or fourth adapter sequence, respectively, and wherein the third and fourth adapter sequences are different from each other;
(e) a reverse transcriptase;
(f) a DNA polymerase; and
(g) deoxy-ribonucleoside triphosphate (dNTP).

Also provided is a method of determining whether a subject is at risk of a particular type of cancer or at risk for a particular type of cancer associated with a particular genotype, the method comprising detecting a gene alteration by the following steps
(a) mixing a target RNA obtained from the biological sample, a template-switching oligo, a reverse transcriptase, and a reverse transcription (RT) primer in a mixture, wherein the template switch oligo comprises a first universal primer sequence and the RT primer comprises a second universal primer sequence;
(b) subjecting the mixture to a condition under which reverse transcription occurs to provide a target DNA complementary to the target RNA,
(c) amplifying the target DNA with a first pair of primers to obtain a first PCR product, wherein the first pair of primers comprise a gene specific primer and a universal primer, the gene specific primer comprises a 5'-end first adapter sequence and a 3'-end sequence that hybridizes to a target gene under stringent conditions, the universal primer comprises a 5'-end second adapter sequence and a 3'-end sequence comprising the first or second universal primer sequence, and the first and second adapter sequences are different from each other;
(d) amplifying the first PCR product with a second pair of primers to obtain a second PCR product, wherein each of the second pair of primers comprises a 5'-end third or fourth adapter sequence and a 3'-end sequence that hybridizes to the first or second adapter sequence, respectively, and the third and fourth adapter sequences are different from each other; and
(e) analyzing the second PCR product to detect the presence of the gene alteration, preferably, the second PCR product is analyzed by sequencing analysis utilizingat least one adapter sequence.

The present disclosure is further illustrated by the following Examples, which are provided for the purpose of demonstration rather than limitation.

### EXAMPLES

### Example 1.

### Detect gene fusion by Targeted RNA sequencing with PCR and template switching technology

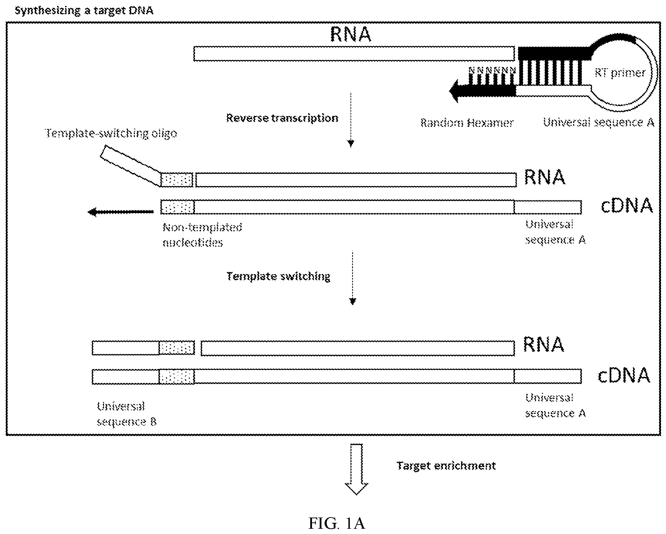

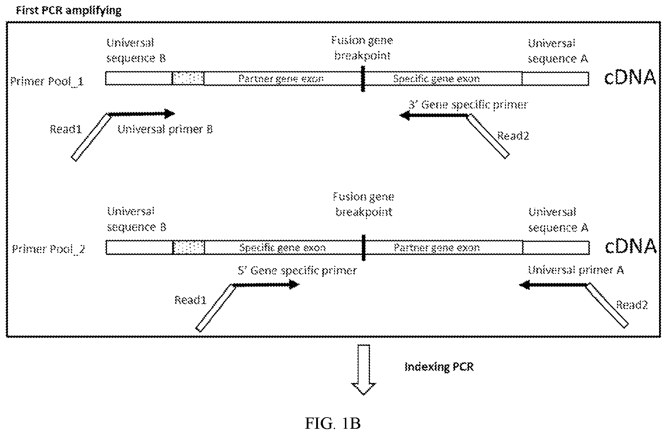

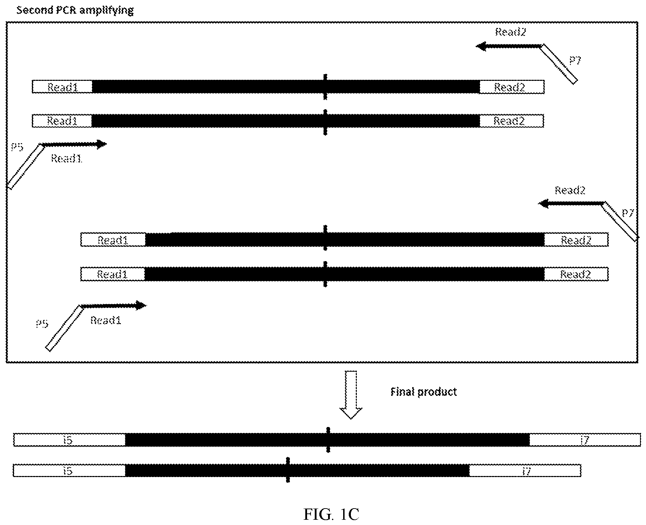

A method was developed to obtain any unknown DNA or RNA sequence, particularly DNA or RNA containing a genetic alteration, such as a gene fusion, next to any targetable sequence, especially from samples of poor quality and low quantity. The overall steps of an exemplary method to detect gene fusions using an RNA sequence as the initial template is shown in Fig. 1. To obtain an unknown sequence, a primer (e.g., from Integrated Device Technology (IDT), Inc.) is used to bind to target RNA fragments and make the target DNA by reverse transcription, followed by PCR amplification with universal primers, and eventually, PCR products containing the unknown sequence will be obtained and sequenced by Sanger method.

For experimental step 1, Formalin-Fixed Paraffin-Embedded (FFPE)-derived RNA sample was denatured at 65°C for 5 minutes, cooled on ice, and then reacted with reverse transcription (RT) random primer containing a universal sequence A at the 5' end of the primer for first strand cDNA synthesis. The reverse transcriptase in the Template Switching RT Enzyme Mix (from New England Biolabs, Inc.) added a few non-templated nucleotides to the 3'-end of the synthesized DNA, after it reaches the 5' end of RNA template. The non-templated nucleotides served as an anchoring site for the template-switching (TS) oligo.

For experimental step 2, the template-switching oligo (TSO) containing a universal primer B was prepared. Upon base pairing between the TS oligo and the non-templated nucleotides, the reverse transcriptase switched template strand, from the RNA to the TS oligo, and continued the extension of the cDNA to the 5' end of the TS oligo, adding the universal sequence B to the 3'-end of the synthesized cDNA. RT cleanup reaction and post-RT purification of the synthesized cDNA were conducted using commercially available kits according to the manufacture's protocol (OmniFusion^{™} RNA).

For experimental step 3, enrichment of target cDNA was conducted by using two primer pools. The primer Pool1 and Pool2 were used for detection of unknown 5' fusion partners and unknown 3' fusion partners, respectively. For detection of unknown 5' fusion partners, the multiplex PCR (mPCR) was performed by using a forward universal primer B connected to Read1 sequence and a reverse 3' gene specific primer connected to Read2 sequence. For detection of unknown 3' fusion partners, the multiplex PCR was performed using the reverse universal primer A connected to Read2 sequence and the forward 5' gene specific primer connected to Read1 sequence. After target enrichment, post-mPCR purification, digestion reaction and post-digestion purification were conducted using commercially available kits according to the manufacture's protocol (OmniFusion^{™} RNA).

For experimental step 4, the adapter sequences (Read1 and Read2) at both ends of the purified DNA from step 3 were used as annealing sites for base pairing with the specific primers with unique illumina adapter sequences for the second PCR amplification (indexing purpose). The second PCR products were then prepared for detecting the fusion events by Sanger-sequencing or next-generation sequencing (such as illumina or ion torrent).

Fig. 1 shows the overall process of this method. The two-step PCR method can successfully enrich the target product of the potential gene fusion present to detect the gene fusion events when the partner gene is unknown or not included on the panel.

The universal primer can be any of the primers listed in Table 1, where each universal primer can be used as either the universal forward primer or the universal reverse primer.

**TABLE 1**

| Universal primer No. | Primer sequence (from the 5' end to the 3' end) | SEQ ID NO |
|---|---|---|
| U01 | GTTTTCCCAGTCACGACGT | 1 |
| U02 | GCAAATGGCATTCTGACATCC | 2 |
| U03 | GCGGATAACAATTTCACACAGG | 3 |
| U04 | CGTCCATGCCGAGAGTG | 4 |
| U05 | CTTTATGTTTTTGGCGTCTTCCA | 5 |
| U06 | GACTGGTTCCAATTGACAAGC | 6 |
| U07 | GCGTGAATGTAAGCGTGAC | 7 |
| U08 | TGTAAAACGACGGCCAGT | 8 |
| U09 | AAGGGTCTTGCGAAGGATAG | 9 |
| U10 | GGGTTATGCTAGTTATTGCTCAG | 10 |

### Example 2.

### Compare PCR product sizes for different RT primers

Based on the design described herein for detecting novel fusion transcripts, gene-specific primers are divided into two separate primer pools, i.e., one for the detection of 5' fusion genes and another for the detection of 3' fusion genes. Figure 2 illustrates the design of the primers that can be used in a method of the application.

To test the effect of different reverse transcription (RT)-related primers on the efficiency and specificity of reverse transcription, three kinds of RT primers containing a universal primer sequence were utilized for the construction of cDNA library by *in vitro* reverse transcription using a mixture of PBMC RNAs as templates. The evaluated RT primers include linear form of random hexamer primers with a universal primer tag on its 5' end, stem-loop form of random hexamer-based primers with a universal primer tag positioned in loop region only, and stem-loop form of random hexamer-based primers with a universal primer tag located in both loop and stem region (Fig. 3).

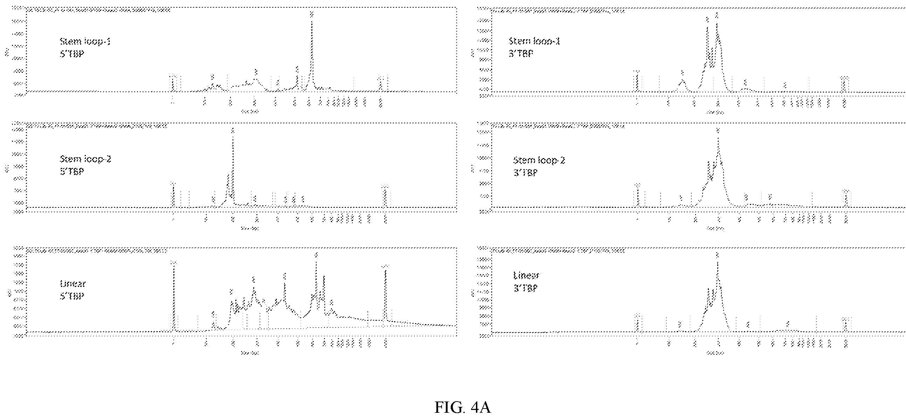

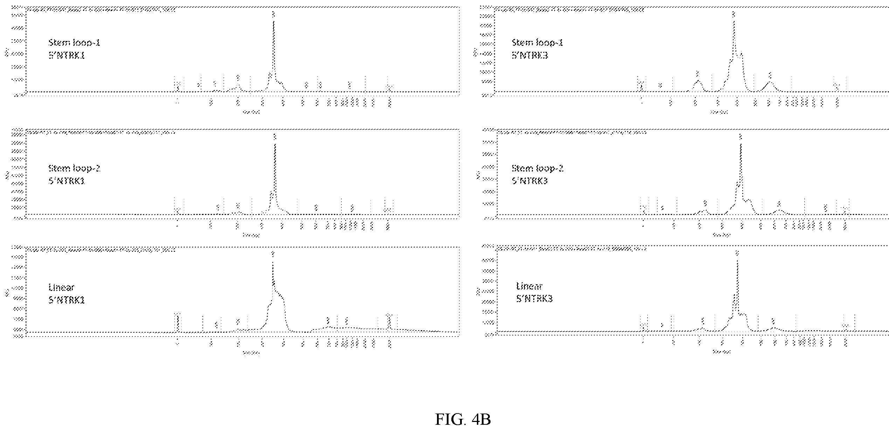

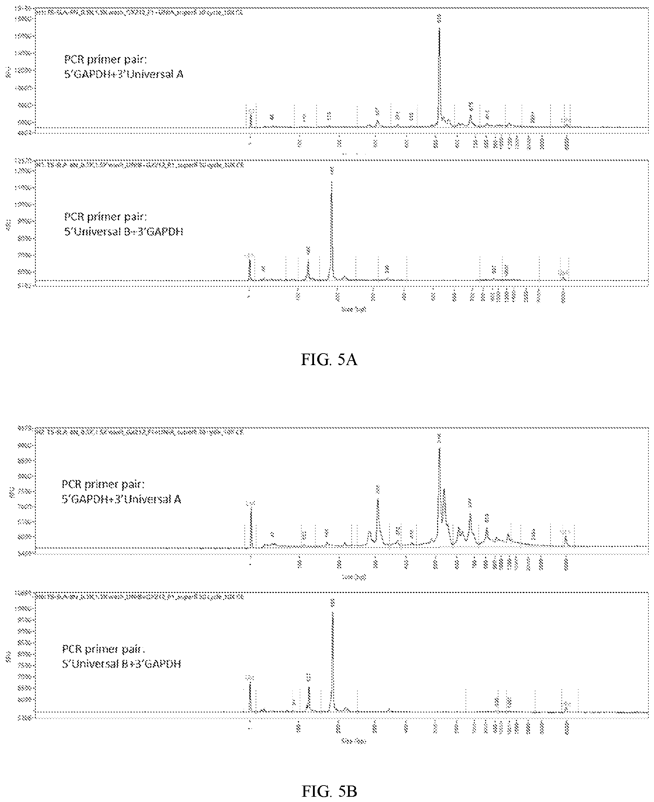

Figures 4a, 4b, 5a, and 5b show the relative fluorescence unit (RFU) values of the PCR products of different sizes when different RT-related primers were used. The results showed that all three kinds of RT primer could successfully synthesize cDNA products for further PCR amplification procedure (Figs. 4a and 4b). The specificity and performance between designed linear random hexamer primers and designed stem-loop primers are different, and the appropriate RT primers can be chosen as the RT conversion oligomers in the library construction system based on the need.

### Example 3.

### Determine the potential MET gene alteration

A 3' or 5' gene specific primer targeting MET exon 14 skipping mutation nucleic acid (shown in Table 2) was designed based on the nucleotide sequence of a known fusion junction sequence in the RNA transcript of the MET gene (as shown in Table 3). The 3' or 5' gene specific primer is utilized to detect gene fusion events encompassing the known fusion junction as well as other fusion types by a method of the application. The first 20 and the last 20 base pairs of the sequence in Table 3 are from the 5' partner (exon 13 of the MET gene) and the 3' partner (exon 15 of the MET gene), respectively. Other 3' or 5' gene specific primer sequences can also be used to detect MET gene fusion events using a method of the application.

**TABLE 2**

| Fusion Type | Sequence of the primer design (from the 5' end to the 3' end) | | SEQ ID NO |
|---|---|---|---|
| MET-MET fusion | 5' | AAGAGAAAGCAAATTAAAGATCAGTT | 11 |
| | 3' | CTGTCAGAGGATACTGCAC | 12 |

**TABLE 3**

| Fusion Type | 5' gene | 3' gene | Sequence of the fusion region (from the 5' end to the 3' end) | SEQ ID NO |
|---|---|---|---|---|
| MET-MET fusion | MET exon 13 | MET exon 15 | AAAGAGAAAGCAAATTAAAG ATCAGTTTCCTAATTCATCT | 13 |

### Example 4.

### Determine the potential NTRK gene alteration

Table 4 shows the various NTRK fusion types and some 3' or 5'-gene specific primers that can be used in a method of the application. A 3' or 5' gene specific primer targeting NTRK exon mutation nucleic acid was designed based on the nucleotide sequence of a fusion region in the RNA transcript of the NTRK gene fusion. The primer is utilized to detect gene fusion events by a method of the application. Other 3' or 5' gene specific primer sequences can also be used to detect NTRK gene fusion events using a method of the application.

**TABLE 4**

| Fusion Type | Sequence of the primer design (from the 5' end to the 3' end) | | SEQ ID NO |
|---|---|---|---|
| TFG-NTRK1 fusion | 5' | ATGTCAGCGTTTGGCTT | 14 |
| | 3' | TTTCGTCCTTCTTCTCCACC | 15 |
| ETV6-NTRK3 fusion | 5' | CCACATCATGGTCTCTGTCT | 16 |
| | 3' | GGCTGAGTCCTCCTCAC | 17 |
| ETV6-NTRK3 fusion | 5' | CAGCCGGAGGTCATACT | 18 |
| | 3' | GGCTGAGTCCTCCTCAC | 19 |
| QKI-NTRK2 fusion | 5' | CCAGCTACATCAATCCTTGAG | 20 |
| | 3' | CTGGCAGAGTCATCATCATT | 21 |
| TFG-NTRK1 fusion | 5' | ACAGCAGCCACCATATACA | 22 |
| | 3' | AGGTGTTTCGTCCTTCTT | 23 |
| TFG-NTRK1 fusion | 5' | TGGCTTAACAGATGATCAGG | 24 |
| | 3' | GAGAAGGGGATGCACCA | 25 |
| TPM3-NTRK1 fusion | 5' | GACCCGTGCTGAGTTTG | 26 |
| | 3' | CAGCCCATCCTCTGGAG | 27 |
| ETV6-NTRK2 fusion | 5' | GAGGTCATACTGCATCAGAAC | 28 |
| | 3' | CATTGGAGATGTGATGGAGTG | 29 |
| ETV6-NTRK3 fusion | 5' | TCCCCGCCTGAAGAGCA | 30 |
| | 3' | TCTCGCTTCAGCACGAT | 31 |
| TFG-NTRK3 fusion | 5' | ACCATATACAGGAGCTCAGAC | 32 |
| | 3' | CTCGATGCAGTGCTCCA | 33 |

### Example 5.

### Determine the potential EGFRvIII gene alteration

A 3' or 5' gene specific primer targeting an EGFR exon mutation nucleic acid (shown in Table 5) was designed based on the nucleotide sequence of a fusion region in the RNA transcript of an EGFR-EGFR fusion gene (as show in Table 6). The 3' or 5' gene specific primer is utilized to detect gene fusion events by a method of the application. Other 3' or 5' gene specific primer sequences can also be used to detect EGFR gene fusion events using a method of the application.

**TABLE 5**

| Fusion Type | Sequence of the primer design (from the 5' end to the 3' end) | | SEQ ID NO |
|---|---|---|---|
| | 5' | GGGCTCTGGAGGAAAAGAA | 34 |
| EGFR-EGFR fusion | 3' | TCCATCTCATAGCTGTCGG | 35 |

**TABLE 6**

| Fusion Type | 5' gene | 3' gene | Sequence of the fusion region (from the 5' end to the 3' end) | SEQ ID NO |
|---|---|---|---|---|
| EGFR-EGFR fusion | EGFR exon 1 | EGFR exon 8 | GCTCTGGAGGAAAAGAAAGT TGTGGTGACAGATCACGGCT | 36 |

## Claims

1. A method for detecting a gene alteration in a biological sample, comprising steps of:
(a) mixing a target RNA obtained from the biological sample, a template-switching oligo, a reverse transcriptase, and a reverse transcription (RT) primer in a mixture, wherein the template switch oligo comprises a first universal primer sequence and the RT primer comprises a second universal primer sequence;
(b) subjecting the mixture to a condition under which reverse transcription occurs to provide a target DNA complementary to the target RNA,
(c) amplifying the target DNA with a first pair of primers to obtain a first PCR product, wherein the first pair of primers comprise a gene specific primer and a universal primer, the gene specific primer comprises a 5'-end first or second adapter sequence and a 3'-end sequence that hybridizes to a target gene under stringent conditions, the universal primer comprises a 5'-end first or second adapter sequence and a 3'-end sequence comprising the first or second universal primer sequence, and the first and second adapter sequences are different from each other;
(d) amplifying the first PCR product with a second pair of primers to obtain a second PCR product, wherein each of the second pair of primers comprises a 5'-end third or fourth adapter sequence and a 3'-end sequence that hybridizes to the first or second adapter sequence, respectively, and the third and fourth adapter sequences are different from each other; and
(e) analyzing the second PCR product to detect the presence of the gene alteration, preferably, the second PCR product is analyzed by sequencing analysis utilizing at least one adapter sequence.

2. The method of claim 1, wherein the RT primer comprises a linear structure having at least 5 random nucleotides, or the RT primer comprises a stem-loop structure and an overhang structure having at least 5 random nucleotides,
preferably wherein the stem-loop structure is a hairpin stem-loop structure or a Y shape stem-loop structure, or the stem-loop structure comprises a barcode sequence, and
more preferably wherein the stem-loop structure is a hairpin stem-loop structure or a Y shape stem-loop structure, and the stem-loop structure comprises a barcode sequence .

3. The method of claim 2, wherein the stem-loop structure is at least the length of the second universal primer sequence or the stem of the stem-loop structure is 8 bp in length, wherein the second universal primer is preferably less than 30 bp in length.

4. The method of any one of claims 1-3, wherein at least one of the first adapter sequence or the second adapter sequence comprises a barcode sequence.

5. The method of any one of claims 1-4, wherein the target DNA is amplified by multiplex PCR with at least two gene specific primers in step (c).

6. The method of claim 1, wherein the first PCR product is generated and separated by 3' or 5' direction of the gene specific primer.

7. The method of claim 1, wherein the gene specific primer *hybridizes* to the target DNA within a distance of at least 25 bp from a fusion junction boundary or
wherein the one or more gene specific primers are selected from the group consisting of SEQ ID NOs:11, 12, 14-35 and any complementary sequence thereof.

8. The method of claim 1, wherein the gene alteration is a gene fusion comprising a sequence of a known gene selected from the group consisting of ABL1, AKT3, ALK, ARV7, BCR, BRAF, CD74, EGFR, ERBB2, ERBB4, ERG, ESR1, ETV1, ETV4, ETV5, ETV6, EZR, FGFR1, FGFR2, FGFR3, KIT, KMT2A, MET, NRG1, NRG2, NTRK1, NTRK2, NTRK3, NUTM1, PDGFRA, PDGFRB, PIK3CA, RAF1, RARA, RET, ROS1, RSPO2, SDC4, SLC34A2 and TMPRSS2.

9. The method of any one of claims 1-8, wherein the biological sample is from a solid tumor or a Formalin-Fixed Paraffin-Embedded (FFPE) tissue sample.

10. The method of any one of claims 1-9, wherein the second PCR product is analyzed by a next generation sequencing.

11. A kit for detecting a gene alteration in a biological sample, comprising:
(a) a template switch oligo comprising a first universal primer;
(b) a reverse transcription (RT) primer comprising a stem-loop structure and an overhang structure of at least 5 random tail nucleotides, wherein the RT primer comprises a second universal primer;
(c) one or more gene specific primers each comprising a first or second adapter sequence and universal primers each comprising a first or second adapter sequence, respectively, wherein the first adapter sequence of the gene specific primer is different from the second adapter sequence of the universal primer or the second adapter sequence of the gene specific primer is different from the first adapter sequence of the universal primer;
(d) a primer pair complementing the first and second adapters, respectively, wherein each primer of the primer pair comprises a third or fourth adapter sequence, respectively, and wherein the third and fourth adapter sequences are different from each other;
(e) a reverse transcriptase;
(f) a DNA polymerase; and
(g) deoxy-ribonucleoside triphosphate (dNTP).

12. A kit for detecting a gene alteration in a biological sample, comprising:
(a) a template switch oligo comprising a first universal primer;
(b) a reverse transcription (RT) primer comprising a linear structure of at least 5 random tail nucleotides, wherein the RT primer comprises a second universal primer;
(c) one or more gene specific primers each comprising a first or second adapter sequence and universal primers each comprising a first or second adapter sequence, respectively, wherein the first adapter sequence of the gene specific primer is different from the second adapter sequence of the universal primer or the second adapter sequence of the gene specific primer is different from the first adapter sequence of the universal primer;
(d) a primer pair complementing the first and second adapters, respectively, wherein each primer of the primer pair comprises a third or fourth adapter sequence, respectively, and wherein the third and fourth adapter sequences are different from each other;
(e) a reverse transcriptase;
(f) a DNA polymerase; and
(g) deoxy-ribonucleoside triphosphate (dNTP).

13. The kit of claim 11, wherein the stem of the stem loop primer is 8 bp in length or the overhang structure is a random hexamer with a length of 5 to 10 nucleotides.

14. The kit of claim 11 or 12, wherein the kit further comprises at least one labeled dNTP, wherein the dNTP is labeled with a biotin group, Digoxigenin (DIG) or other molecules.

## Patentansprüche

1. Verfahren zum Nachweisen einer Genveränderung in einer biologischen Probe, umfassend die Schritte:
(a) Mischen einer aus der biologischen Probe erhaltenen Ziel-RNA, ein Template-Switching-Oligonucleotid, eine Reverse Transkriptase und einen Primer für Reverse Transkription (RT) in einem Gemisch, wobei das Template-Switching-Oligonucleotid eine erste universelle Primersequenz umfasst und der RT-Primer eine zweite universelle Primersequenz umfasst;
(b) Herstellen von Bedingungen für das Gemisch, unter denen eine reverse Transkription stattfindet, wobei man eine Ziel-DNA erhält, die komplementär zu der Ziel-RNA ist,
(c) Amplifizieren der Ziel-DNA mit einem ersten Paar von Primern, wobei man ein erstes PCR-Produkt erhält, wobei das erste Paar von Primern einen genspezifischen Primer und einen universellen Primer umfasst, der genspezifische Primer eine erste oder zweite 5'-Ende-Adaptersequenz und eine 3'-Ende-Sequenz, die unter stringenten Bedingungen mit einem Ziel-Gen hybridisiert, umfasst, der universelle Primer eine erste oder zweite 5'-Ende-Adaptersequenz und eine 3'-Ende-Sequenz, die die erste oder zweite universelle Primersequenz umfasst, umfasst und die erste und die zweite Adaptersequenz voneinander verschieden sind;
(d) Amplifizieren des ersten PCR-Produkts mit einem zweiten Paar von Primern, wobei man ein zweites PCR-Produkt erhält, wobei jeder aus dem zweiten Paar von Primern eine dritte oder vierte 5'-Ende-Adaptersequenz und eine 3'-Ende-Sequenz, die mit der ersten bzw. zweiten Adaptersequenz hybridisiert, umfasst und die dritte und die vierte Adaptersequenz voneinander verschieden sind; und
(e) Analysieren des zweiten PCR-Produkts unter Nachweis der Anwesenheit der Gen-Veränderung, wobei vorzugsweise das zweite PCR-Produkt durch Sequenzierungsanalyse unter Verwendung wenigstens einer Adaptersequenz analysiert wird.

2. Verfahren gemäß Anspruch 1, wobei der RT-Primer eine lineare Struktur mit wenigstens 5 zufälligen Nucleotiden umfasst oder der RT-Primer eine Stamm-Schleife-Struktur und eine Überhangstruktur mit wenigstens 5 zufälligen Nucleotiden umfasst,
wobei vorzugsweise die Stamm-Schleife-Struktur eine Haarnadel-Stamm-Schleife-Struktur oder eine Y-Form-Stamm-Schleife-Struktur ist oder die Stamm-Schleife-Struktur eine Strichcodesequenz umfasst und
wobei besonders bevorzugt die Stamm-Schleife-Struktur eine Haarnadel-Stamm-Schleife-Struktur oder eine Y-Form-Stamm-Schleife-Struktur ist und die Stamm-Schleife-Struktur eine Strichcodesequenz umfasst.

3. Verfahren gemäß Anspruch 2, wobei die Stamm-Schleife-Struktur wenigstens die Länge der zweiten universellen Primersequenz aufweist oder der Stamm der Stamm-Schleife-Struktur eine Länge von 8 bp aufweist,
wobei der zweite universelle Primer vorzugsweise eine Länge von weniger als 30 bp aufweist.

4. Verfahren gemäß einem der Ansprüche 1-3, wobei die erste Adaptersequenz und/oder die zweite Adaptersequenz eine Strichcodesequenz umfasst.

5. Verfahren gemäß einem der Ansprüche 1-4, wobei die Ziel-DNA in Schritt (c) durch Multiplex-PCR mit wenigstens zwei genspezifischen Primern amplifiziert wird.

6. Verfahren gemäß Anspruch 1, wobei das erste PCR-Produkt in der 3'- oder 5'-Richtung des genspezifischen Primers erzeugt und abgetrennt wird.

7. Verfahren gemäß Anspruch 1, wobei der genspezifische Primer innerhalb eines Abstands von wenigstens 25 bp von einer Fusionsverknüpfungsgrenze mit der Ziel-DNA hybridisiert oder
wobei der eine oder die mehreren genspezifischen Primer aus der Gruppe ausgewählt sind, die aus SEQ ID Nr. 11, 12, 14-35 und jeder dazu komplementären Sequenz besteht.

8. Verfahren gemäß Anspruch 1, wobei die Genveränderung eine Genfusion ist, die eine Sequenz eines bekannten Gens umfasst, das aus der Gruppe ausgewählt ist, die aus ABL1, AKT3, ALK, ARV7, BCR, BRAF, CD74, EGFR, ERBB2, ERBB4, ERG, ESR1, ETV1, ETV4, ETV5, ETV6, EZR, FGFR1, FGFR2, FGFR3, KIT, KMT2A, MET, NRG1, NRG2, NTRK1, NTRK2, NTRK3, NUTM1, PDGFRA, PDGFRB, PIK3CA, RAF1, RARA, RET, ROS1, RSPO2, SDC4, SLC34A2 und TMPRSS2 besteht.

9. Verfahren gemäß einem der Ansprüche 1-8, wobei die biologische Probe aus einem soliden Tumor oder einer Formalin-fixierten, in Paraffin eingebetteten (FFPE) Gewebeprobe stammt.

10. Verfahren gemäß einem der Ansprüche 1-9, wobei das zweite PCR-Produkt durch eine Sequenzierung der nächsten Generation analysiert wird.

11. Kit zum Nachweisen einer Genveränderung in einer biologischen Probe, umfassend:
(a) ein Template-Switching-Oligonucleotid, das einen ersten universellen Primer umfasst;
(b) einen Primer für Reverse Transkription (RT), der eine Stamm-Schleife-Struktur und eine Überhangstruktur mit wenigstens 5 zufälligen Schwanz-Nucleotiden umfasst, wobei der RT-Primer einen zweiten universellen Primer umfasst;
(c) ein oder mehrere genspezifische Primer, die jeweils eine erste oder zweite Adaptersequenz umfassen, bzw. universelle Primer, die jeweils eine erste oder zweite Adaptersequenz umfassen, wobei sich die erste Adaptersequenz des genspezifischen Primers von der zweiten Adaptersequenz des universellen Primers unterscheidet oder sich die zweite Adaptersequenz des genspezifischen Primers von der ersten Adaptersequenz des universellen Primers unterscheidet;
(d) ein Primerpaar, das zu dem ersten bzw. dem zweiten Adapter komplementär ist, wobei jeder Primer des Primerpaars eine dritte bzw. vierte Adaptersequenz umfasst und wobei die dritte und die vierte Adaptersequenz voneinander verschieden sind;
(e) eine Reverse Transkriptase;
(f) eine DNA-Polymerase; und
(g) Desoxyribonucleosidtriphosphat (dNTP).

12. Kit zum Nachweisen einer Genveränderung in einer biologischen Probe, umfassend:
(a) ein Template-Switching-Oligonucleotid, das einen ersten universellen Primer umfasst;
(b) einen Primer für Reverse Transkription (RT), der eine lineare Struktur mit wenigstens 5 zufälligen Schwanz-Nucleotiden umfasst, wobei der RT-Primer einen zweiten universellen Primer umfasst;
(c) einen oder mehrere genspezifische Primer, die jeweils eine erste oder zweite Adaptersequenz umfassen, bzw. universelle Primer, die jeweils eine erste oder zweite Adaptersequenz umfassen, wobei sich die erste Adaptersequenz des genspezifischen Primers von der zweiten Adaptersequenz des universellen Primers unterscheidet oder sich die zweite Adaptersequenz des genspezifischen Primers von der ersten Adaptersequenz des universellen Primers unterscheidet;
(d) ein Primerpaar, das zu dem ersten bzw. dem zweiten Adapter komplementär ist, wobei jeder Primer des Primerpaars eine dritte bzw. vierte Adaptersequenz umfasst und wobei die dritte und die vierte Adaptersequenz voneinander verschieden sind;
(e) eine Reverse Transkriptase;
(f) eine DNA-Polymerase; und
(g) Desoxyribonucleosidtriphosphat (dNTP).

13. Kit gemäß Anspruch 11, wobei der Stamm der Stamm-Schleife-Struktur eine Länge von 8 bp aufweist oder die Überhangstruktur ein zufälliges Hexamer mit einer Länge von 5 bis 10 Nucleotiden ist.

14. Kit gemäß Anspruch 11 oder 12, wobei der Kit weiterhin wenigstens ein markiertes dNTP umfasst, wobei das dNTP mit einer Biotingruppe, Digoxigenin (DIG) oder anderen Molekülen markiert ist.

## Revendications

1. Procédé pour détecter une modification génétique dans un échantillon biologique, comprenant les étapes consistant à :
(a) mélanger un ARN cible obtenu dudit échantillon biologique, un oligonucléotide de commutation de matrice, une transcriptase inverse, et une amorce pour transcription reverse (RT) dans un mélange, dans lequel ledit oligonucléotide de commutation de matrice comprend une première séquence d'amorce universelle, et ladite amorce pour RT comprend une deuxième séquence d'amorce universelle,
(b) soumettre le mélange à une condition sous laquelle une transcription reverse se fait pour procurer un ADN cible complémentaire à l'ARN cicle,
(c) amplifier l'ADN cible avec une première paire d'amorces pour obtenir un premier produit de PCR, dans lequel la première paire d'amorces comprend une amorce génospécifique et une amorce universelle, l'amorce génospécifique comprend une première ou deuxième séquence adaptatrice du terminus 5', et une séquence du terminus 3' qui s'hybride à un gène cible sous des conditions rigoureuses, l'amorce universelle comprend une première ou deuxième séquence adaptatrice du terminus 5', et une séquence du terminus 3' comprenant la première ou deuxième séquence d'amorce universelle, et lesdites première et deuxième séquences adaptatrices sont différentes l'une de l'autre,
(d) amplifier le premier produit de PCR avec une deuxième paire d'amorces pour obtenir un deuxième produit de PCR, dans lequel chacune de ladite deuxième paire d'amorces comprend une troisième ou quatrième séquence adaptatrice du terminus 5', et une séquence du terminus 3' qui s'hybride à la première ou deuxième séquence adaptatrice, respectivement, et lesdites troisième et quatrième séquences adaptatrices sont différentes l'une de l'autre, et
(e) analyser le deuxième produit de PCR pour détecter la présence de la modification génétique, de préférence le deuxième produit de PCR est analysé par analyse par séquençage en utilisant au moins une séquence adaptatrice.

2. Procédé selon la revendication 1, dans lequel l'amorce de RT comprend une structure linéaire ayant au moins 5 nucléotides aléatoires, ou l'amorce de RT comprend une structure tige-boucle et une structure excédentaire ayant au moins 5 nucléotides aléatoires,
de préférence dans lequel ladite structure tige-boucle est une structure tige-boucle en épingle à cheveux ou une structure tige-boucle sous forme d'Y, ou la structure tige-boucle comprend une séquence de code à barres, et
de préférence encore dans lequel ladite structure tige-boucle est une structure tige-boucle en épingle à cheveux ou une structure tige-boucle sous forme d'Y, et la structure tige-boucle comprend une séquence de code à barres.

3. Procédé selon la revendication 2, dans lequel ladite structure tige-boucle a une longueur au moins égale à celle de la deuxième séquence d'amorce universelle, ou la tige de ladite structure tige-boucle a une longueur de 8 bp,
dans lequel la deuxième amorce universelle de préférence a une longueur de moins de 30 bp.

4. Procédé selon l'une quelconque des revendications 1-3, dans lequel au moins l'une parmi la première séquence adaptatrice ou la deuxième séquence adaptatrice comprend une séquence de code à barres.

5. Procédé selon l'une quelconque des revendications 1-4, dans lequel l'ADN cible est amplifié par PCR multiplexe avec au moins deux amorces génospécifiques dans l'étape (c).

6. Procédé selon la revendication 1, dans lequel le premier produit de PCR est généré et séparé dans la direction 3' ou 5' de l'amorce génospécifique.

7. Procédé selon la revendication 1, dans lequel l'amorce génospécifique s'hybride à l'ADN cible à une distance d'au moins 25 bp d'une limite de jonction de fusion, ou
dans lequel lesdites une ou plusieurs amorces génospécifiques sont choisies dans le groupe consistant en SEQ ID no 11, 12, 14-35 et toute séquence complémentaire à celles-ci.

8. Procédé selon la revendication 1, dans lequel ladite modification génétique est une fusion de gènes comprenant une séquence d'un gène connu choisi dans le groupe consistant à ABL1, AKT3, ALK, ARV7, BCR, BRAF, CD74, EGFR, ERBB2, ERBB4, ERG, ESR1, ETV1, ETV4, ETV5, ETV6, EZR, FGFR1, FGFR2, FGFR3, KIT, KMT2A, MET, NRG1, NRG2, NTRK1, NTRK2, NTRK3, NUTM1, PDGFRA, PDGFRB, PIK3CA, RAF1, RARA, RET, ROS1, RSPO2, SDC4, SLC34A2 et TMPRSS2.

9. Procédé selon l'une quelconque des revendications 1-8, dans lequel ledit échantillon biologique provient d'une tumeur solide, ou est un échantillon de tissu fixé au formol et inclus dans la paraffine (FFIP).

10. Procédé selon l'une quelconque des revendications 1-9, dans lequel le deuxième produit de PCR est analysé par séquençage de la prochaine génération.

11. Trousse pour détecter une modification génétique dans un échantillon biologique, comprenant :
(a) un oligonucléotide de commutation de matrice comprenant une première amorce universelle,
(b) une amorce pour transcription reverse (RT) comprenant une structure tige-boucle et une structure excédentaire ayant au moins 5 nucléotides de queue aléatoires, où ladite amorce pour RT comprend une deuxième amorce universelle,
(c) une ou plusieurs amorces génospécifiques, comprenant chacune une première ou deuxième séquence adaptatrice, et des amorces universelles comprenant chacune une première ou deuxième séquence adaptatrice, respectivement, dans lequel la première séquence adaptatrice de l'amorce génospécifique est différente de la deuxième séquence adaptatrice de l'amorce universelle, ou la deuxième séquence adaptatrice de l'amorce génospécifique est différente de la première séquence adaptatrice de l'amorce universelle,
(d) une paire d'amorces complémentaires au premier et deuxième adapteurs, respectivement, dans lequel chaque amorce de la paire d'amorces comprend une troisième ou quatrième séquence adaptatrice, respectivement, et dans lequel lesdites troisième et quatrième séquences adaptatrices sont différentes l'une de l'autre,
(e) une transcriptase reverse,
(f) une polymérase d'ADN, et
(g) du triphosphate de désoxyribonucléoside (dNTP).

12. Trousse pour détecter une modification génétique dans un échantillon biologique, comprenant :
(a) un oligonucléotide de commutation de matrice comprenant une première amorce universelle,
(b) une amorce pour transcription reverse (RT) comprenant une structure linéaire d'au moins 5 nucléotides de queue aléatoires, où ladite amorce pour RT comprend une deuxième amorce universelle,
(c) une ou plusieurs amorces génospécifiques, comprenant chacune une première ou deuxième séquence adaptatrice, et des amorces universelles comprenant chacune une première ou deuxième séquence adaptatrice, respectivement, dans lequel la première séquence adaptatrice de l'amorce génospécifique est différente de la deuxième séquence adaptatrice de l'amorce universelle, ou la deuxième séquence adaptatrice de l'amorce génospécifique est différente de la première séquence adaptatrice de l'amorce universelle,
(d) une paire d'amorces complémentaires au premier et deuxième adapteurs, respectivement, dans lequel chaque amorce de la paire d'amorces comprend une troisième ou quatrième séquence adaptatrice, respectivement, et dans lequel lesdites troisième et quatrième séquences adapttrices sont différentes l'une de l'autre,
(e) une transcriptase reverse,
(f) une polymérase d'ADN, et
(g) du triphosphate de désoxyribonucléoside (dNTP).

13. Trousse selon la revendication 11, dans laquelle la tige de ladite amorce tige-boucle a une longueur de 8 bp, ou la structure excédentaire est un hexamère aléatoire avec une longueur de 5 à 10 nucléotides.

14. Trousse selon la revendication 11 ou 12, dans laquelle la trousse comprend en outre au moins un dNTP marqué, dans lequel le dNTP est marqué avec un groupe biotine, du digoxigénine (DIG), ou d'autres molécules.
